Europäisches Patentamt

European Patent Office

Office européen des brevets

(19)

(11) Numéro de publication:

**0 242 305**
**A1**

(12) **DEMANDE DE BREVET EUROPEEN**

(21) Numéro de dépôt: 87400900.4

(22) Date de dépôt: 17.04.87

(51) Int. Cl.⁴: **C 12 N 5/00,** A 61 K 37/12, A 61 L 15/00

(30) Priorité: 18.04.86 FR 8605591

(71) Demandeur: **INSTITUT MERIEUX Société anonyme, 19, rue Bourgelat, F-69002 Lyon (FR)**

(43) Date de publication de la demande: **21.10.87 Bulletin 87/43**

(72) Inventeur: **Tayot, Jean-Louis, 1 rue des Greffières, F-69890 Le tour de Salvagny (FR)**
Inventeur: **Nguyen, Cuc, 6 Chemin du Grand-Chêne, F-69260 Charbonnieres-les-Bains (FR)**
Inventeur: **Tardy, Michel, 165 bis rue Joliot Curie, F-69005 Lyon (FR)**

(84) Etats contractants désignés: **AT BE CH DE ES GB GR IT LI NL SE**

(74) Mandataire: **Lemoine, Michel et al, Cabinet Michel Lemoine et Bernasconi 13 Boulevard des Batignolles, F-75008 Paris (FR)**

(54) **Procédé de culture microbiologique sur supports de collagène, supports pour ce procédé et produits obtenus.**

(57) Procédé de culture microbiologique sur supports de collagène, caractérisé en ce que l'on constitue un gel, stable à pH neutre et à une température de culture cellulaire, notamment environ 37°C, à partir de collagène placentaire de type I et/ou III, ayant subi une étape de digestion enzymatique et que l'on amène des cellules à coloniser ce support.

EP 0 242 305 A1

Procédé de culture microbiologique sur supports de collagène, supports pour ce procédé et produits obtenus.

La présente invention a trait à un procédé de culture microbiologique, et notamment de culture de cellules, sur support de collagène, ainsi qu'à des supports pour ce procédé de culture. L'invention a également trait aux produits nouveaux obtenus par ce procédé.

Le développement des systèmes de cultures cellulaires tri-dimensionnelles est en plein essor et permet d'envisager de nombreuses applications en médecine et dans les biotechnologies.

Devant le problème posé par la reconstitution de la barrière cutanée des grands brûlés, on a cherché, en dehors de la mise en place d'autogreffes, disponibles en quantités insuffisantes chez les grands brûlés, ou d'allogreffes, se traduisant généralement par des phénomènes de rejet, à recouvrir la peau de nappes ou films synthétiques ou reconstitués à partir de collagène.

En conséquence, depuis plusieurs années, les

2

recherches se sont orientées vers la mise au point d'une peau artificielle produite in vitro.

Le collagène, qui constitue le composant majeur de la matrice extra-cellulaire in vivo, a été largement étudié en gel tri-dimensionnel pour les cultures de cellules. Cela a été en particulier le cas pour les cultures hematopoïétiques ; voir LANOTTE M. Terminal differentiation of hemopoietic cell clones cultured in tridimensional collagen matrix : in situ cell morphology and enzyme histochemistry analysis, Biol. Cell, 50, 107-120, 1984 ; pour des cellules folliculaires thyroïdiennes, voir CHAMBARD M., VERRIER B, GABRION J. and MAUCHAMP J. Polarity reversal of inside-out thyroid follicles cultured within collagen gel : reexpression of specific functions, Biol. Cell, 51, 315-326, 1984 ; et surtout pour les fibroblastes cutanés d'après le modèle de BELL. pour la production d'un derme équivalent, voir COULOMB B, DUBERTRET L., BELL E., MERRIL C., FOSSE M., BRETON-GORIUS J. PROST C. and TOURAINE R. Endogenous peroxydases in normal human dermis : a marker of fibroblast differentiation, J. Invest. Dermatol., 81, (1), 75-78, 1983, et COULOMB B. DUBERTRET L., BELL E. and TOURAINE R. The contractility of fibroblasts in a collagen lattice is reduced by corticosteroids, J. Invest. Dermatol., 82 (4) 341-344, 1984.

D'après ces derniers auteurs, seul un collagène de type I non pepsiné peut donner des propriétés rétractiles en présence de fibroblastes pour former un réseau manipulable de bonne solidité mécanique.

Un tel derme équivalent et son procédé de fabrication sont décrits dans le brevet US-A-4 485 096 et dans la demande de brevet WO 80/01350. Ces documents décrivent un derme artificiel humain obtenu en mélangeant du collagène provenant de la peau ou des tendons de veau ou de rat, c'est-à-dire de collagène animal de type I, avec des cellules de fibroblastes humains en présence d'un milieu de culture convenable. Le procédé consiste à former un mélange conduisant à un gel de collagène habité par des fibroblastes qui s'attachent aux fibres et provoquent l'agencement d'une structure en réseau. Le derme artificiel ainsi obtenu peut ensuite être ensemencé en surface avec des cellules épithéliales ou kératinocytes prélevés sur le brûlé lui-même.

Ces développements, basés sur l'utlisation du collagène sont d'une grande importance en médecine, pour le traitement des brûlés, le développement de nouveaux pansements, d'agents cicatrisants et de biomatériaux.

On comprend donc l'importance de disposer de collagènes identiques aux collagènes humains ou s'en rapprochant le plus possible, si l'on veut éviter toute réaction inflammatoire et immunologique. Or les seules préparations de collagène disponibles commercialement en quantités suffisantes et à prix acceptable, à ce jour, sont obtenus à partir de queues ou tendons de rats ou à partir de peau de jeunes animaux (veaux, porcs) et sont exclusivement constitués de collagène de type I. De telles préparations présentent cependant l'inconvénient de comporter des risques de réactions inflammatoires et immunologiques pour l'organisme récepteur.

Il est, d'autre part, abondamment publié que, pour réaliser des cultures cellulaires tri-dimensionnelles, et notamment de fibroblastes cutanés, pour la réalisation de dermes artificiels, il est indispensable d'utiliser du collagène acido-soluble de rats ou de veaux n'ayant pas subi de dégradation enzymatique. Voir FREY et al. 1er Congrès Atlantique sur le Collagène, Lyon. Septembre (1985). De telles préparations de collagène animal acido-soluble contenant leurs télopeptides, sont très immunogènes, ce qui peut être un inconvénient sérieux pour le développement de leurs applications en médecine humaine.

Il serait donc désirable de pouvoir utiliser des collagènes d'origine humaine mais l'isolement de collagène humain n'est réalisable, sur le plan pratique et

4

industriel, qu'à partir de placenta. Voir les brevets français 81.22606 et 85.13004. Ces documents, ainsi que d'autres documents publiés sur ce sujet, attestent de l'impossibilité pratique d'isoler des quantités significative de collagène humain placentaire acido-soluble, sans recourir à une digestion enzymatique ou chimique pour couper les télopeptides et autres liens inter-moléculaires, cette digestion étant réalisée, dans la pratique, en utilisant la pepsine en milieu acide.

Or, la littérature précitée affirme que ces traitements de digestion du collagène le rendent impropre à servir de support pour la culture tri-dimensionnelle de cellules.

Le brevet français 81 22606 qui recourt à un traitement alcalin du collagène placentaire prévoit d'effectuer éventuellement une solubilisation à la pepsine du collagène ainsi traité. Le collagène ainsi obtenu ne constitue pas un support de culture convenable pour des cultures cellulaires.

La présente invention a cependant permis de montrer, de façon surprenante, qu'il était possible, à partir de collagènes humains placentaires traités par un procédé enzymatique de digestion connu, notamment à la pepsine, de former des gels constitués de fibres insolubles dans l'eau à pH neutre et stables à une température comprise entre 25 et 40°C, notamment à 37°C et qui soient habitables par des cellules et notamment des fibroblastes, en donnant les propriétés de rétraction déjà décrites avec le collagène animal non-pepsiné.

La présente invention se propose de fournir un procédé de culture microbiologique sur supports de collagène, permettant l'usage à l'échelle industrielle, de collagène humain d'origine placentaire.

Un autre objectif de l'invention est de fournir un tel procédé permettant d'obtenir des propriétés rétractiles en présence de cellules, notamment de fibroblastes, pour former un réseau manipulable de bonne qualité mécanique.

Un autre objectif de l'invention est de fournir un tel procédé de culture tri-dimensionnelle permettant de former des nappes à base de collagènes.

Un autre objectif encore de l'invention est de fournir un tel procédé qui permette de réaliser des cultures en suspension, applicables à la culture de cellules ainsi qu'à la culture de virus sur cellules.

L'invention a pour objet un procédé de culture micro-biologique sur supports de collagène, caractérisé en ce que l'on constitue un support en un gel, stable à pH neutre et à une température de culture cellulaire, de préférence environ 37°C, à partir de collagène placentaire de type I et/ou III, ayant subi une étape de digestion enzymatique, et que l'on amène des cellules à coloniser ce support.

Dans un mode de mise en oeuvre préféré de l'invention, le support est réalisé à partir d'un gel de collagène pepsiné en milieu acide.

L'invention a également pour objet les supports réalisés en un tel gel et destinés à être colonisés par les cellules lors du procédé de culture, lesdits supports pouvant recevoir des formes diverses.

En particulier, ces supports peuvent être réalisés sous forme de nappes.

Ces supports peuvent cependant également être réalisés sous forme de particules de faibles dimensions, par exemple des billes, permettant, en utilisant de tels micro-supports, de réaliser des cultures cellulaires sur les micro-supports en suspension dans un fermenteur.

Il est ainsi possible d'obtenir, grâce à l'invention, des produits tels que, notamment, des nappes et des particules ou billes, de préférence de très petite taille susceptibles d'être utilisées, notamment, à titre de produits cicatrisants.

6

Les micro-supports selon l'invention peuvent également être utilisés pour des cultures virales ainsi que pour la production de vaccins.

D'autres avantages et caractéristiques de l'invention apparaîtront à la lecture de la description suivante, faite à titre d'exemple non limitatif.

Exemple 1.

Procédé de préparation d'un collagène placentaire humain de type I + III.

300 kg de placenta humain sont broyés sous forme congelée pour donner des morceaux de quelques cm3. Le broyat est ensuite mélangé à 300 l d'une solution aqueuse contenant, au stade final, 8 % d'éthanol, 6 g/l de NaCl et 10 kg de cellulose. Après agitation à 10°C, l'ensemble est soumis à une opération de pressage avec un pressoir pour séparer le sang du tissu placentaire. On obtient ainsi 102 kg de tissu placentaire contenant 65 % d'eau.

Le tissu extrait du pressoir est agité dans 500 l de citrate de sodium 0,05 M à pH 7,2 pendant 30 mn à 10°C puis soumis au pressage pour récupérer le tissu. Un second lavage est effectué avec 500 l de cette même solution additionnée de 30 g/l de NaCl. Après récupération du tissu par pressage, un troisième lavage est effectué avec 500 l de citrate de sodium 0,05 M à pH 7,2. De préférence, le tissu ainsi lavé à pH neutre est ensuite soumis à plusieurs séquences de lavage à pH acide, toujours à 10°C.

Ces lavages peuvent être effectués dans des solutions d'acide citrique à pH acide 2,8 par addition d'HCl, puis d'acide formique 0,5 M pendant environ 15 heures et puis d'acide citrique additionné de NaCl à 20 g/l.

Le tissu placentaire ainsi lavé à pH acide et récupéré après pressage, a un aspect blanc qui témoigne d'une bonne élimination des pigments de sang placentaire initial. Le poids obtenu est de 82 kg.

Le tissu est soumis à une digestion enzymatique par la pepsine dans 500 l d'acide citrique 0,05 M à pH

7

2,8 contenant 300 g de pepsine pendant 15 heures à +10°C.

La suspension est ensuite diluée par addition de 500 l d'eau à +10°C et le pH ajusté à 7,5. Après 15 heures d'attente à +10°C le résidu tissulaire qui contient encore l'essentiel des collagènes I et III est séparé sur centrifugeuse. On obtient 103 kg de résidus.

Ce résidu est repris dans 800 l d'acide citrique 0,05 M à pH 2,8 contenant 300 g de pepsine, pendant 72 heures à +10°C. La suspension est alors diluée par addition de 200 l de NaCl à 50 g/l avant élimination du résidu tissulaire sur centrifugeuse.

Le pH du surnageant est ajusté à 7,5 par addition de soude pour dénaturer la pepsine. Après 2 heures d'attente, le pH est réajusté à 2,7 par addition de HCL 4N et laissé pendant 15 heures à +4°C, après quoi, on centrifuge la suspension. Le précipité est éliminé et le surnageant est additionné de NaCl 30 g/l. Après 15 heures à +4°C, le précipité de collagène formé est récupéré par centrifugation. On obtient 12 kg de précipité qui sont redissouts dans 600 l d'HCl 0,01 M et ensuite dilués avec 600 l de NaCl 24 g/l. Après 15 heures à +4°C, le précipité est éliminé par centrifugation et le surnageant est additionné de NaCl jusqu'à une concentration finale de 30 g/l.

On laisse se reposer pendant 15 heures à +4°C puis le précipité de collagène est recueilli par centrifugation. On récupère 8 kg de précipité.

Le précipité fait ensuite l'objet de plusieurs lavages à l'acétone puis est séché sous courant d'air stérile permettant d'obtenir 1 kg de fibres sèches et stériles de collagène contenant un mélange des types I et III.

Exemple 2.

Préparation d'un gel .

La poudre formée par les fibres sèches obtenues dans l'exemple 1 est remise en solution en eau stérile à une concentration de 2 g/l. On mélange cette

solution de collagène à un milieu de culture concentré DMEM (DULBECCO modified EAGLE Medium) pour obtenir une suspension homogénéisée dont la teneur est la suivante :

Milieu de culture DMEM 5 x c 1 ml

H2O 0,55 ml

Sérum de veau foetal (SVF) 0,45 ml

Collagène 2 mg/ml 2,5 ml.

Cette suspension peut ensuite être coulée très doucement dans un récipient destiné à lui donner la forme voulue.

Exemple 3.

On coule très doucement la suspension obtenue dans l'exemple 2 dans une boîte Falcon d'un diamètre de 5 cm dont le fond est tapissé d'une mono-couche confluente de fibroblastes et on complète avec 0,5 ml de milieu nutritif DMEM enrichi de 10 % de sérum de veau foetal.

Le gel de collagène polymérise rapidement à pH neutre et à 37°C.

On maintient la culture ainsi réalisée à température de 37°C sous atmosphère enrichie en $CO_2$ et l'on change périodiquement le milieu de culture.

On constate que la couche de gel de collagène se rétracte progressivement en épaisseur mais ne subit aucune rétraction en diamètre . Au bout de 4 jours, on obtient un film de collagène habité par les fibroblastes et dont l'épaisseur est de 1 mm. Le diamètre de ce film reste stable, comme le montre la courbe H sur le graphique. Ce film constitue une nappe qui présente des qualités mécaniques suffisantes pour pouvoir être manipulée et utilisée comme derme équivalent.

Exemple 4.

On reprend l'exemple 3 avec cette différence qu'avant de couler dans la boîte déjà tapissée de la couche confluente de cellules, la suspension destinée à fournir la couche de gel de collagène, on ajoute à la suspension 0,5 ml de milieu DMEM contenant 200.000 cellules MRC-5 trypsinées en milieu DMEM à 10 % de sérum de veau foetal.

Comme on le voit sur la courbe G du graphique, la culture se poursuit sans aucune rétraction en diamètre. Seule l'épaisseur de la nappe diminue comme dans l'exemple 3.

Exemple 5.

On réalise un gel de collagène ayant la composition prévue à l'exemple 2 que l'on laisse polymériser de façon à obtenir une couche ou nappe de gel ayant une épaisseur de 3 mm.

On prépare séparément, dans une boîte Falcon, une couche confluente de cellules MRC-5 entretenues en milieu DMEM à 10 % de sérum de veau foetal. Une fois la couche confluente obtenue, on transvase délicatement la couche de collagène gélifiée de façon à l'amener en superposition sur la couche confluente de cellules.

On constate que les cellules se mettent à coloniser le collagène et il se produit une rétraction en épaisseur de la nappe de gel de collagène, l'épaisseur se stabilisant au bout de 7 jours à environ 0,5 mm. Aucune rétraction en diamètre n'a lieu.

Exemple 6.

Dans une boîte Falcon d'un diamètre de 5 cm, on met en place une suspension ayant la composition décrite à l'exemple 2 et on reprend des cellules MRC-5 trypsinées en milieu DMEM à 10 % de sérum de veau foetal. On ajoute 0,5 ml de milieu contenant les cellules à la suspension destinée à former le gel. On homogénéise la solution obtenue et on incube à 37°C sous $CO_2$. On évalue périodiquement la contraction en diamètre du gel. On constate que, comme on le voit sur la courbe A, le diamètre de 5 cm se trouve réduit à environ 1 cm au bout de huit jours.

Si, sur une autre boîte, préparée de façon identique, on recouvre le gel tel qu'il vient d'être défini à la fin du premier jour, par une deuxième couche de gel, contenant également des fibroblastes MRC-5, on obtient une courbe de rétraction en diamètre (courbe C) pratiquement analogue à la courbe A. De même, si dans une troisième boîte on superpose à la fin du premier jour une deuxième couche ne contenant cette fois pas de fibroblastes, on obtient également, comme le montre la courbe B, une rétraction progressive en diamètre comparable à l'allure de la courbe A.

Exemple 7.

On prépare une boîte Falcon avec une suspension de collagène contenant des fibroblastes comme dans l'exemple 6.

A la fin du premier jour, on transvase le gel sur une mono-couche confluente ; on constate que la rétraction en diamètre s'arrête rapidement comme montre le courbe F. Seule la rétraction en épaisseur se poursuit.

Exemple 8.

On reprend l'exemple 7 et, immédiatement après avoir transvasé le gel sur la couche confluente, on recouvre le gel d'un deuxième gel contenant des fibroblastes et on observe une courbe de rétraction représentée en D dont le diamètre se stabilise rapidement.

Exemple 9.

Dans le cas où, à la différence de l'exemple 8, le second gel de recouvrement ne contient pas de fibroblastes, on observe aussi, comme le montre la courbe E, un arrêt rapide de la rétraction en diamètre.

Exemple 10.

On reproduit les résultats décrits dans les exemples 3 à 9 avec un gel de collagène placentaire ne contenant que des protéines humaines en remplacement du sérum de veau foetal.

La composition d gel est la suivante :

| | | |
|---|---|---|
| DMEM 5 x c | 1 | ml |
| H2O | 0,45 | ml |
| Albumine humaine 50 mg/ml | 0,5 | ml |
| Serum humain | 0,05 | ml |
| Collagène I + III 2 mg/ml | 2,5 | ml |

11

Les cellules trypsinées, reprises en DMEM + 10 % SVF, sont centrifugées 1 mn à 1000 g à +4°C. Ensuite, on reprend le culot cellulaire en milieu DMEM sans sérum de veau. On dépose 0,5 ml de la suspension cellulaire (200.000 cellules) dans la suspension de collagène.

L'ensemble est coulé dans des boîtes de 5 cm de diamètre selon les conditions décrites dans les exemples précités ; les résultats sont identiques.

Exemple 11.

On reprend l'exemple 10, avec un gel de collagène placentaire appauvri davantage en protéines humaines.

La composition du gel est la suivante :

| DMEM 5 x c | 1 ml |
| H2O | 0,95 ml |
| Serum humain | 0,05 ml |
| Collagène I + III (2 mg/ml) | 2,5 ml |

Les résultats sont identiques.

Exemple 12.

On reprend l'exemple 3 en utilisant une mono-couche confluente de fibroblastes cutanés développée à partir d'une biopsie de peau humaine, à la place des cellules MRC 5.

Les résultats sont identiques.

Exemple 13.

On reprend l'exemple 3 en utilisant une mono-couche de chondrocytes de cartilage humain, à la place des cellules MRC 5.

Les résultats sont identiques.

Exemple 14.

Préparation de micro-billes de collagène.

Dans un mélange de 9 volumes d'acétone et de 1 volume de NaOH 0,1 N, avec l'aide d'une seringue équipée d'une aiguille 27 G ou 25 G, on dépose en goutte à goutte une solution acide de collagène placentaire I + III à 2 % réalisée à partir de la poudre de collagène selon l'exemple

1.

Chaque gouttelette se gélifie immédiatement et conserve définitivement sa forme sphérique.

La suspension obtenue est ensuite lavée par une solution stérile physiologique pour éliminer l'acétone et neutraliser le pH de manière définitive. Les micro-billes obtenues ont un diamètre de l'ordre de 1 mm.

Elles peuvent ensuite être utilisées en culture cellulaire.

D'autres procédés peuvent être utilisés pour réaliser de grandes quantités de micro-billes de collagène. En particulier, on peut utiliser tous les procédés connus pour préparer des émulsions de micro-gouttelettes. Ainsi, on peut réaliser une émulsion dont l'une des deux phases est constituée par la solution acide de collagène précitée et on peut prévoir un agent alcalin dans l'autre phase ou ajouter progressivement un agent alcalin à l'ensemble pour neutraliser l'acidité du collagène et le gélifier aux environs de la neutralité.

Exemple 15.

Culture cellulaire sur micro-billes de collagène placentaire.

Des billes de 1 mm de diamètre réalisées dans l'exemple précédent sont mises dans une suspension de fibroblastes humains diploïdes MRC 5 en milieu DMEM (DULBECCO MODIFIED EAGLE MEDIUM) contenant 10 % de SVF à 37°C, sous agitation.

Après 2 heures, la totalité des fibroblastes est fixée à la surface des billes. Après 4 jours de culture à 37°C, le volume total des billes est colonisé par les fibroblastes qui ont réalisé une contraction importante du collagène puisque le diamètre des billes est devenu finalement de l'ordre de 100 à 200 microns.

Une telle suspension de micro--billes habitées par les fibroblastes peut être utilisée, par exemple dans la culture des virus.

0242305

13

On a effectué des cultures similaires avec des fibroblastes cutanés et avec des chondrocytes du cartilage avec des résultats similaires.

14

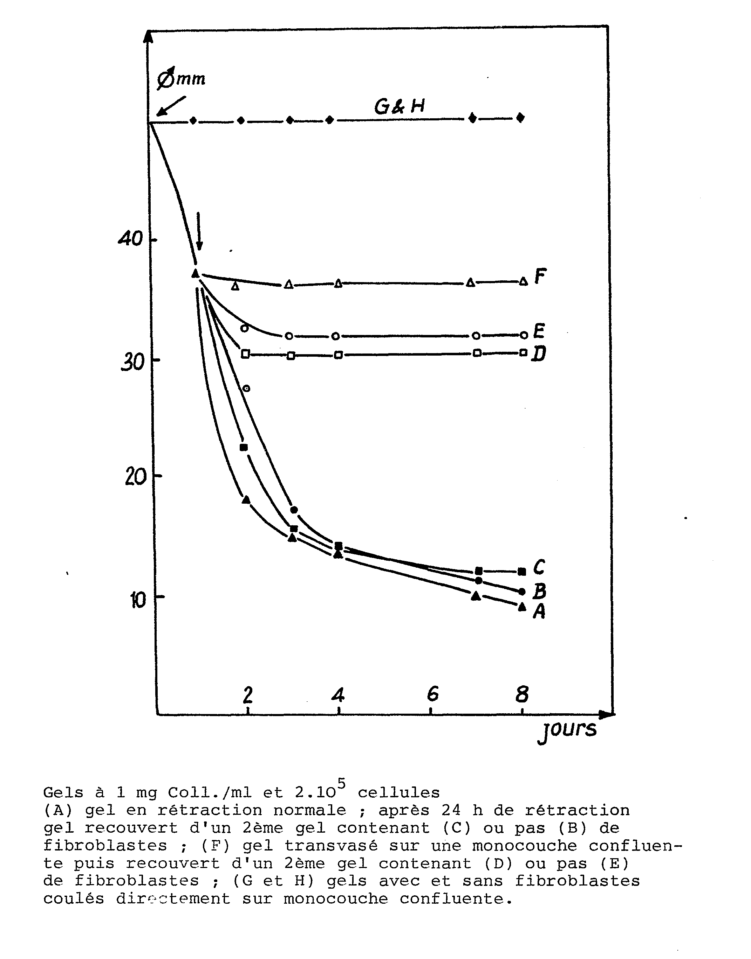

REVENDICATIONS

1. Procédé de culture microbiologique sur supports de collagène, caractérisé en ce que l'on constitue un gel, stable à pH neutre et à une température de culture cellulaire, notamment environ 37°C, à partir de collagène placentaire de type I et/ou III, ayant subi une étape de digestion enzymatique et que l'on amène des cellules à coloniser ce support.

2. Procédé selon la revendication 1, caractérisé en ce que l'on réalise le support à partir d'un gel de collagène pepsiné en milieu acide.

3. Procédé selon l'une quelconque des revendications 1 et 2, caractérisé en ce que l'on constitue une nappe de collagène.

4. Procédé selon l'une des revendications 1 et 2, caractérisé en ce que l'on constitue des micro-particules de collagène, notamment en forme de billes.

5. Procédé selon l'une quelconque des revendications 1 à 4, caractérisé en ce que l'on introduit des cellules lors de la formation du gel, en milieu nutritif convenable.

6. Procédé selon l'une quelconque des revendications 1 à 5, caractérisé en ce que l'on amène des cellules en contact avec le support dans un milieu nutritif convenable.

7. Procédé selon les revendications 4 et 6, caractérisé en ce que l'on disperse des micro-particules, notamment des billes, de collagène dans un milieu nutritif convenable en présence de cellules en suspension.

8. Procédé selon l'une quelconque des revendications 1 à 7, caractérisé en ce que l'on utilise un milieu de culture DMEM.

9. Procédé selon la revendication 8, caractérisé en ce que l'on effectue la culture en présence de sérum de veau foetal.

10. Procédé selon la revendication 8, caractérisé en ce que l'on effectue la culture en présence d'albumine humaine.

11. Procédé selon l'une quelconque des revendications 1 à 10, caractérisé en ce que l'on effectue une culture de fibroblastes, notamment MRC 5.

12. Procédé l'une quelconque des revendications 1 à 10, caractérisé en ce que l'on effectue une culture de chondrocytes.

13. Support de collagène pour procédé de culture micro-biologique, caractérisé en ce qu'il est réalisé à partir d'un gel, stable à pH neutre et à 37°C, obtenu à partir de collagène placentaire de type I et/ou III, ayant subi une étape de digestion enzymatique ou chimique, notamment à la pepsine.

14. Support selon la revendication 13, caractérisé en ce qu'il se présente sous forme d'une nappe.

15. Support selon la revendication 13, caractérisé en ce qu'il se présente sous forme de micro-particules et notamment de billes.

16. Support selon la revendication 15, caractérisé en ce que le diamètre initial des micro-particules est de l'ordre de 1 mm.

17. Application des supports selon l'une quelconque des revendications 13 à 16 à la culture de virus sur cellule.

18. Application des supports selon l'une des revendications 15 et 16 à la formation de pansements.

Gels à 1 mg Coll./ml et $2.10^5$ cellules
(A) gel en rétraction normale ; après 24 h de rétraction
gel recouvert d'un 2ème gel contenant (C) ou pas (B) de
fibroblastes ; (F) gel transvasé sur une monocouche confluente puis recouvert d'un 2ème gel contenant (D) ou pas (E)
de fibroblastes ; (G et H) gels avec et sans fibroblastes
coulés directement sur monocouche confluente.

0242305

**RAPPORT DE RECHERCHE EUROPEENNE**

Numero de la demande

EP  87 40 0900

Office européen
des brevets

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| X,D | EP-A-0 080 956 (FONDATION MERIEUX) <br> * Page 6, lignes 5-14,29-36; page 7, lignes 1,2; page 8, lignes 24-36; page 9, lignes 1-5,30-35; page 10, lignes 6-12; page 11, lignes 4-8; revendications * | 13,14 | C 12 N 5/00 <br> A 61 K 37/12 <br> A 61 L 15/00 |
| Y | | 1-12, 15-17 | |
| Y | DIE ANGEWANDTE MAKROMOLEKULARE CHEMIE, vol. 111, no. 1701, 1983, pages 107-122, Hüthig & Wepf Verlag, Basel, CH; M.Z. ABEDIN et al.: "Collagen heterogeneity and its functional significance" <br> * Page 110 * | 1-12, 15-17 | |
| | | | **DOMAINES TECHNIQUES RECHERCHES (Int. Cl.4)** |
| A | IDEM. | 13,14 | C 12 N <br> A 61 K <br> A 61 L |
| Y | EP-A-0 119 076 (KOKEN CO. LTD.) <br> * En entier * | 4,7,15 -17 | |

Le présent rapport de recherche a été établi pour toutes les revendications

| Lieu de la recherche | Date d'achèvement de la recherche | Examinateur |
|---|---|---|
| LA HAYE | 16-06-1987 | SKELLY J.M. |